# EUROPÄISCHE PATENTANMELDUNG

(11) **EP 4 668 141 A1**
(43) Veröffentlichungstag der Anmeldung: **24.12.2025**
(21) Anmeldenummer: 25182307.6
(22) Anmeldetag: 12.06.2025
(51) Int. Cl.: G06F 21/31, H04L 9/40

(54) **STEUERN EINES ZUGRIFFS AUF EIN MEDIZINTECHNISCHES GERÄT**

(30) Priorität: 17.06.2024 DE 102024116911
(71) Anmelder: Löwenstein Medical Technology S.A., 2557 Luxembourg (LU)
(72) Erfinder: Dordevic, Milos, 22305 Hamburg (DE)
(74) Vertreter: Löwenstein Medical IP

(57) **Zusammenfassung**

Ein Verfahren zum Ermöglichen eines Benutzerzugriffs auf ein medizintechnisches Gerät (7) über ein Benutzergerät (9) umfasst folgende Schritte, die durch einen Prozessor (3a) des medizintechnischen Geräts (7) ausgeführt werden: Generieren eines verschlüsselten Passworts (13) durch Verschlüsseln eines zu verschlüsselnden Passworts (12); Senden des verschlüsselten Passworts (13) ans Benutzergerät (9), um ein Entschlüsseln des verschlüsselten Passworts (13) zu ermöglichen; Empfangen einer vom Benutzergerät (9) gesendeten Zugriffsanfrage (19) zum Anfordern des Benutzerzugriffs, wobei die Zugriffsanfrage (19) ein entschlüsseltes Passwort (17) umfasst; Prüfen, ob das entschlüsselte Passwort (17) mit dem zu verschlüsselnden Passwort (12) übereinstimmt; wenn das entschlüsselte Passwort (17) mit dem zu verschlüsselnden Passwort (12) übereinstimmt: Ermöglichen des Benutzerzugriffs.

## Beschreibung

### Technisches Gebiet

Die Erfindung betrifft ein Verfahren zum Ermöglichen eines Benutzerzugriffs auf ein medizintechnisches Gerät über ein Benutzergerät. Des Weiteren betrifft die Erfindung ein Verfahren zum Anfordern eines Benutzerzugriffs auf ein medizintechnisches Gerät über ein Benutzergerät, ein Verfahren zum Betreiben eines Entschlüsselungsgeräts und ein Verfahren zum Steuern eines Benutzerzugriffs auf ein medizintechnisches Gerät über ein Benutzergerät. Darüber hinaus betrifft die Erfindung eine Vorrichtung zur Datenverarbeitung, ein Computerprogramm und ein computerlesbares Medium zum Ausführen mindestens eines dieser Verfahren.

### Stand der Technik

Auf das Betriebssystem eines modernen medizintechnischen Geräts wie z. B. eines Beatmungsgeräts kann in der Regel über ein externes Benutzergerät wie z. B. einen PC oder einen Laptop zugegriffen werden, beispielsweise um eine Hard- und/oder Softwarekonfiguration des medizintechnischen Geräts zu ändern und/oder bestimmte Funktionen des medizintechnischen Geräts zu testen. Um das medizintechnische Gerät vor unbefugten Benutzern zu schützen, kann der Zugriff mit einem gerätetypspezifischen Passwort beschränkt sein. Die Änderung eines solchen Passworts, beispielsweise im Fall eines Passwortdiebstahls, kann mit einem gewissen Aufwand verbunden sein, insbesondere dann, wenn eine Vielzahl medizintechnischer Geräte betroffen ist.

### Offenbarung der Erfindung

Eine Aufgabe der Erfindung kann darin gesehen werden, ein Verfahren zu schaffen, das eine verbesserte Steuerung eines Benutzerzugriffs auf ein medizintechnisches Gerät über ein Benutzergerät ermöglicht. Eine weitere Aufgabe der Erfindung kann darin gesehen werden, eine entsprechende Vorrichtung zur Datenverarbeitung, ein entsprechendes Computerprogramm und ein entsprechendes computerlesbares Medium bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Verfahren zum Ermöglichen eines Benutzerzugriffs auf ein medizintechnisches Gerät über ein Benutzergerät. Das Verfahren wird durch einen Prozessor des medizintechnischen Geräts ausgeführt und umfasst: Generieren eines verschlüsselten Passworts durch Verschlüsseln eines zu verschlüsselnden Passworts (beispielsweise aus einem Speicher des medizintechnischen Geräts); Senden des verschlüsselten Passworts ans Benutzergerät, um ein (externes) Entschlüsseln des verschlüsselten Passworts zu ermöglichen; (anschließend:) Empfangen einer vom Benutzergerät gesendeten Zugriffsanfrage zum Anfordern des Benutzerzugriffs, wobei die Zugriffsanfrage ein entschlüsseltes Passwort umfasst; Prüfen, ob das entschlüsselte Passwort mit dem zu verschlüsselnden Passwort übereinstimmt; wenn das entschlüsselte Passwort mit dem zu verschlüsselnden Passwort übereinstimmt: Ermöglichen des Benutzerzugriffs auf das medizintechnische Gerät über das Benutzergerät.

Dadurch, dass das Passwort nur in verschlüsselter Form durch das medizintechnische Gerät bereitgestellt werden kann und erst durch ein externes Gerät entschlüsselt werden muss, kann das Risiko ungewollter Zugriffe auf das medizintechnische Gerät im Vergleich zu einer Ausführungsform ohne eine derartige Ver- bzw. Entschlüsselung - beispielsweise einer Ausführungsform, bei der das medizintechnische Gerät mit einem gerätetypspezifischen Passwort aus einer zentralen Passwortdatenbank geschützt wird - deutlich reduziert werden.

Bei dem medizintechnischen Gerät kann es sich allgemein um ein Medizinprodukt mit einem Prozessor oder einem Computer, beispielsweise in Form eines eingebetteten Systems, handeln. Beispielsweise kann das medizintechnische Gerät ein Beatmungsgerät zum invasiven und/oder nicht invasiven Beatmen eines Patienten, ein Hustengerät zum Unterstützen eines Patienten beim Husten, ein Überwachungsmonitor zum Überwachen von Vitalparametern eines Patienten, ein Defibrillator, ein Herzschrittmacher, ein Hörgerät, ein Gerät der bildgebenden Diagnostik oder ein Operationsroboter sein.

Unter "Benutzergerät" kann vor- und nachstehend ein externer Computer - beispielsweise ein PC, ein Server, ein Laptop, ein Tablet oder ein Smartphone - zur Datenkommunikation mit dem medizintechnischen Gerät und einem Entschlüsselungsgerät, wie es nachstehend beschrieben wird, verstanden werden. Dementsprechend kann das Benutzergerät zusätzlich zum Prozessor mindestens eine der folgenden Komponenten umfassen: einen Speicher, ein Bussystem zur Datenkommunikation zwischen dem Speicher und dem Prozessor, eine Datenkommunikationsschnittstelle zur drahtlosen und/oder drahtgebundenen Datenkommunikation mit Peripheriegeräten (beispielsweise über das Internet).

Ein zweiter Aspekt der Erfindung betrifft ein Verfahren zum Anfordern eines Benutzerzugriffs auf ein medizintechnisches Gerät über ein Benutzergerät. Das Verfahren wird durch einen Prozessor des Benutzergeräts ausgeführt und umfasst: Empfangen eines vom medizintechnischen Gerät gesendeten verschlüsselten Passworts; Generieren einer das verschlüsselte Passwort umfassenden Passwortanfrage zum Anfordern eines entschlüsselten Passworts; Senden der Passwortanfrage an ein Entschlüsselungsgerät zum Entschlüsseln des verschlüsselten Passworts; (anschließend:) Empfangen eines vom Entschlüsselungsgerät gesendeten entschlüsselten Passworts; Generieren einer das entschlüsselte Passwort umfassenden Zugriffsanfrage zum Anfordern des Benutzerzugriffs; Senden der Zugriffsanfrage ans medizintechnische Gerät.

Ein dritter Aspekt der Erfindung betrifft ein Verfahren zum Betreiben eines Entschlüsselungsgeräts. Das Verfahren wird durch einen Prozessor des Entschlüsselungsgeräts ausgeführt und umfasst: Empfangen einer von einem Benutzergerät gesendeten Passwortanfrage zum Anfordern eines entschlüsselten Passworts, wobei die Passwortanfrage ein verschlüsseltes Passwort umfasst, wobei das verschlüsselte Passwort durch Verschlüsseln eines zu verschlüsselnden Passworts durch einen Prozessor eines medizintechnischen Geräts generiert wurde; Generieren eines entschlüsselten Passworts durch Entschlüsseln des verschlüsselten Passworts; Senden des entschlüsselten Passworts ans Benutzergerät.

Unter "Entschlüsselungsgerät" kann vor- und nachstehend ein weiterer externer Computer - beispielsweise ein weiterer PC, ein weiterer Server, ein weiterer Laptop, ein weiteres Tablet oder ein weiteres Smartphone - zur Datenkommunikation mit dem Benutzergerät verstanden werden. Dementsprechend kann das Entschlüsselungsgerät zusätzlich zum Prozessor mindestens eine der folgenden Komponenten umfassen: einen Speicher, ein Bussystem zur Datenkommunikation zwischen dem Speicher und dem Prozessor, eine Datenkommunikationsschnittstelle zur drahtlosen und/oder drahtgebundenen Datenkommunikation mit Peripheriegeräten (beispielsweise über das Internet). Das Entschlüsselungsgerät kann auf Hard- und/oder Softwareebene gegen ungewollte Zugriffe besonders gut gesichert sein.

Ein vierter Aspekt der Erfindung betrifft ein Verfahren zum Steuern eines Benutzerzugriffs auf ein medizintechnisches Gerät über ein Benutzergerät. Das Verfahren umfasst die Schritte des vor- und nachstehend beschriebenen Verfahrens gemäß dem ersten Aspekt der Erfindung und die Schritte des vor- und nachstehend beschriebenen Verfahrens gemäß dem zweiten Aspekt der Erfindung. Zusätzlich kann das Verfahren die Schritte des vor- und nachstehend beschriebenen Verfahrens gemäß dem dritten Aspekt der Erfindung umfassen.

Ein fünfter Aspekt der Erfindung betrifft eine Vorrichtung zur Datenverarbeitung. Die Vorrichtung umfasst mindestens eine der folgenden Komponenten:
- einen ersten Prozessor für ein medizintechnisches Gerät, wobei der erste Prozessor konfiguriert ist, um das vor- und nachstehend beschriebene Verfahren gemäß dem ersten Aspekt der Erfindung auszuführen;
- einen zweiten Prozessor für ein Benutzergerät, wobei der zweite Prozessor konfiguriert ist, um das vor- und nachstehend beschriebene Verfahren gemäß dem zweiten Aspekt der Erfindung auszuführen;
- einen dritten Prozessor für ein Entschlüsselungsgerät, wobei der dritte Prozessor konfiguriert ist, um das vor- und nachstehend beschriebene Verfahren gemäß dem dritten Aspekt der Erfindung auszuführen.

Zusätzlich kann die Vorrichtung mindestens eine der folgenden Komponenten umfassen: einen Speicher, ein Bussystem zur Datenkommunikation zwischen dem Speicher und dem jeweiligen Prozessor, eine Datenkommunikationsschnittstelle zur drahtlosen und/oder drahtgebundenen Datenkommunikation mit Peripheriegeräten (beispielsweise über das Internet).

Je nach Ausführungsform kann die Vorrichtung ein einzelner Computer oder eine Kombination mehrerer einzelner Computer (z. B. in einem Computernetzwerk) sein.

Es wird darauf hingewiesen, dass Merkmale der vor- und nachstehend beschriebenen Verfahren auch Merkmale der Vorrichtung sein können (und umgekehrt).

Weitere Aspekte der Erfindung betreffen ein Computerprogramm und ein computerlesbares Medium, auf dem das Computerprogramm gespeichert ist.

Das Computerprogramm umfasst mindestens einen der folgenden Befehlssätze:
- einen ersten Satz von Befehlen, die die vor- und nachstehend beschriebene Vorrichtung (beispielsweise deren ersten Prozessor) beim Ausführen des Computerprogramms durch die Vorrichtung veranlassen, das vor- und nachstehend beschriebene Verfahren gemäß dem ersten Aspekt der Erfindung auszuführen;
- einen zweiten Satz von Befehlen, die die vor- und nachstehend beschriebene Vorrichtung (beispielsweise deren zweiten Prozessor) beim Ausführen des Computerprogramms durch die Vorrichtung veranlassen, das vor- und nachstehend beschriebene Verfahren gemäß dem zweiten Aspekt der Erfindung auszuführen;
- einen dritten Satz von Befehlen, die die vor- und nachstehend beschriebene Vorrichtung (beispielsweise deren dritten Prozessor) beim Ausführen des Computerprogramms durch die Vorrichtung veranlassen, das vor- und nachstehend beschriebene Verfahren gemäß dem dritten Aspekt der Erfindung auszuführen.

Das computerlesbare Medium kann ein flüchtiger oder nicht flüchtiger Datenspeicher sein. Beispielsweise kann das computerlesbare Medium eine Festplatte, ein USB-Speichergerät (*universal serial bus*), ein RAM (*random-access memory*), ein ROM (*read-only memory*), ein EPROM (*erasable programmable read-only memory*), ein EEPROM (*electrically erasable programmable read-only memory*), ein Flash-Speicher oder eine Kombination von mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale der vor- und nachstehend beschriebenen Verfahren auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen. Gemäß einer Ausführungsform kann das verschlüsselte Passwort unter Verwendung eines in einem Speicher des medizintechnischen Geräts gespeicherten öffentlichen Schlüssels generiert werden. Der öffentliche Schlüssel kann mit einem privaten Schlüssel zum Generieren des entschlüsselten Passworts (durch Entschlüsseln eines verschlüsselten Passworts) ein Schlüsselpaar bilden. Das verschlüsselte Passwort kann ausschließlich mithilfe des zugehörigen privaten Schlüssels entschlüsselbar sein.

Das Schlüsselpaar kann zur Verwendung in einem symmetrischen und/oder asymmetrischen Verschlüsselungsverfahren geeignet sein. Es ist möglich, dass das Schlüsselpaar auf einem vom medizintechnischen Gerät getrennten Computer, beispielsweise dem Entschlüsselungsgerät, generiert wird. Der öffentliche Schlüssel kann dann in einen Speicher des medizintechnischen Geräts geschrieben werden, beispielsweise bei der Herstellung des medizintechnischen Geräts. Hingegen kann der private Schlüssel in einem externen Speicher außerhalb des medizintechnischen Geräts und/oder des Benutzergeräts, beispielsweise in einem Speicher des Entschlüsselungsgeräts, gespeichert sein. In einem solchen externen Speicher kann der private Schlüssel zusätzlich in geeigneter Weise geheim gehalten werden, d. h. vor Zugriffen durch Unbefugte geschützt sein. Anders ausgedrückt ist es möglich, dass der private Schlüssel weder mit dem medizintechnischen Gerät noch mit dem Benutzergerät (noch mit einem sonstigen externen Gerät) in irgendeiner Weise ausgetauscht wird. Beispielsweise kann der gleiche öffentliche Schlüssel im Speicher verschiedener medizintechnischer Geräte gespeichert werden, wobei jedes medizintechnische Gerät einen Prozessor zum Ausführen des vor- und nachstehend beschriebenen Verfahrens gemäß dem ersten Aspekt der Erfindung umfassen kann. Somit generiert jedes medizintechnische Gerät sein eigenes Passwort, das nur dem jeweiligen Gerät bekannt ist und nur in verschlüsselter Form durch das jeweilige Gerät bereitgestellt wird.

Gemäß einer Ausführungsform kann das verschlüsselte Passwort in einem asymmetrischen oder hybriden (d. h. sowohl symmetrischen als auch asymmetrischen) Verschlüsselungsverfahren unter Verwendung des öffentlichen Schlüssels generiert werden. Das asymmetrische Verschlüsselungsverfahren, das auch als Public-Key-Verfahren bezeichnet werden kann, kann beispielsweise ein auf der Faktorisierung ganzer Zahlen basierender Algorithmus wie beispielsweise der RSA-Algorithmus (RSA = Rivest-Shamir-Adleman), ein auf dem diskreten Logarithmusproblem und/oder dem Diffie-Hellman-Problem basierender Algorithmus wie beispielsweise der Elgamal-Algorithmus, ein auf elliptischen Kurven basierender Algorithmus oder eine Kombination von mindestens zwei dieser Algorithmen sein. Alternativ sind auch hash-, code- oder gitterbasierte oder multivariate quadratische asymmetrische Algorithmen möglich. Solche Algorithmen sind selbst mit einem Quantencomputer nicht oder nur sehr schwer angreifbar und können daher auch als Post-Quanten-Algorithmen bezeichnet werden. Dies ermöglicht eine besonders sichere Verschlüsselung des Passworts. Das Risiko ungewollter Zugriffe auf das medizintechnische Gerät kann somit auf ein Minimum reduziert werden.

Möglich ist auch eine Generierung des verschlüsselten Passworts in einem symmetrischen Verschlüsselungsverfahren, beispielsweise mit einem DES-Algorithmus (DES = Data Encryption Standard), insbesondere einem 3DES-Algorithmus, und/oder einem AES-Algorithmus (AES = Advanced Encryption Standard). Der 3DES-Algorithmus, d. h. eine Dreifachverschlüsselung mit DES, auch Triple-DES genannt, ist im Vergleich zur Einfachverschlüsselung mit DES besonders sicher, ohne dass die Schlüssellänge übermäßig zunimmt.

Gemäß einer Ausführungsform kann ferner das zu verschlüsselnde (beispielsweise unverschlüsselte) Passwort durch den Prozessor des medizintechnischen Geräts generiert werden. Dazu kann eine bestimmte, beispielsweise zufällige Zeichenfolge generiert und als das zu verschlüsselnde Passwort in einem Speicher des medizintechnischen Geräts gespeichert werden. Dadurch kann eine aufwendige Passwortverwaltung in einer zentralen Datenbank entfallen. Auch kann eine derartige dezentrale Bereitstellung des Passworts das Risiko eines Passwortdiebstahls im Vergleich zu einer zentralen Bereitstellung (beispielsweise mithilfe einer Passwortdatenbank) deutlich verringern, weil das Passwort nur dem jeweiligen medizintechnischen Gerät bekannt ist und von diesem nur in verschlüsselter Form bereitgestellt wird.

Gemäß einer Ausführungsform kann als Reaktion auf das Generieren des zu verschlüsselnden Passworts ein Timer gestartet werden. Wenn der Timer abgelaufen ist, kann bestimmt werden, dass das zu verschlüsselnde Passwort in seiner aktuellen Version ungültig ist, sodass basierend auf der aktuellen Version kein Benutzerzugriff mehr auf das medizintechnische Gerät über das Benutzergerät möglich ist. Dadurch kann das Risiko ungewollter Zugriffe im Vergleich zu einer Ausführungsform mit unbeschränkt gültigem Passwort deutlich reduziert werden. Der Timer kann so konfiguriert sein, dass er nach einer vorgegebenen Dauer von beispielsweise höchstens einer Stunde, höchstens einem Tag, höchstens einer Woche, höchstens einem Monat oder höchstens einem Jahr abläuft.

Gemäß einer Ausführungsform kann - wenn der Timer abgelaufen ist - eine von der aktuellen Version abweichende neue Version des zu verschlüsselnden Passworts generiert werden, die dann statt der aktuellen Version gültig ist. Beispielsweise kann die aktuelle Version mit der neuen Version überschrieben werden. Dies ermöglicht eine automatische Erneuerung des zu verschlüsselnden Passworts in regelmäßigen Abständen. Somit brauchen im Fall eines Passwortdiebstahls keine zusätzlichen - unter Umständen sehr aufwendigen - Sicherungsmaßnahmen getroffen zu werden. Eine derartige Erneuerung des zu verschlüsselnden Passworts kann beispielsweise auch dann erfolgen, wenn das medizintechnische Gerät ausgeschaltet oder in einen Stand-by-Betrieb geschaltet ist.

Gemäß einer Ausführungsform kann der Timer als Reaktion auf das Generieren der neuen Version zurückgesetzt und erneut gestartet werden. Dementsprechend kann bestimmt werden, dass die neue Version ungültig ist, wenn der Timer nach dem erneuten Starten abgelaufen ist, sodass basierend auf der neuen Version kein Benutzerzugriff mehr auf das medizintechnische Gerät über das Benutzergerät möglich ist. Anders ausgedrückt ist es möglich, dass jedes neu generierte Passwort nur für eine bestimmte Dauer gültig ist. Damit kann das Risiko ungewollter Zugriffe weiter reduziert werden.

Gemäß einer Ausführungsform kann das zu verschlüsselnde Passwort jedes Mal neu generiert werden, wenn das medizintechnische Gerät ein- oder ausgeschaltet wird oder zwischen verschiedenen Betriebsarten zum Betreiben des medizintechnischen Geräts, beispielsweise einem Stand-by-Betrieb und einem normalen (Haupt-)Betrieb, umschaltet. Dies ermöglicht eine regelmäßige Erneuerung des Passworts ohne Verwendung eines Timers.

Gemäß einer Ausführungsform kann das zu verschlüsselnde Passwort unter Verwendung eines Zufallsgenerators zum Generieren einer zufälligen Zeichenfolge generiert werden. Dadurch kann sichergestellt werden, dass kein signifikanter Zusammenhang zwischen verschiedenen Versionen des zu verschlüsselnden Passworts besteht. Beispielsweise kann die zufällige Zeichenfolge als das zu verschlüsselnde Passwort in einem Speicher des medizintechnischen Geräts gespeichert werden. Alternativ kann das zu verschlüsselnde Passwort zusätzlich zur zufälligen Zeichenfolge eine nicht zufällige, vorbestimmte Zeichenfolge umfassen.

Gemäß einer Ausführungsform kann die Zugriffsanfrage ferner eine einem Benutzer des Benutzergeräts eindeutig zugeordnete Benutzerkennung umfassen. Beispielsweise kann die Benutzerkennung eine digitale Signatur zum eindeutigen Identifizieren des Benutzers gegenüber dem medizintechnischen Gerät umfassen und/oder bestimmte Zugriffsrechte des Benutzers definieren. Anhand der Benutzerkennung kann geprüft werden, ob der Benutzer zugriffsberechtigt ist. Dementsprechend ist es möglich, dass die Zugriffsanfrage nur dann weiterverarbeitet wird oder der Benutzerzugriff auf das medizintechnische Gerät nur dann ermöglicht wird, wenn der Benutzer zugriffsberechtigt ist.

Denkbar ist auch, dass eine solche Benutzerkennung vor dem Senden des verschlüsselten Passworts ans Benutzergerät im medizintechnischen Gerät empfangen wird, beispielsweise vom Benutzergerät und/oder von einem mobilen Datenträger wie z. B. einem USB-Stick. Dementsprechend ist es möglich, dass das verschlüsselte Passwort nur dann ans Benutzergerät gesendet wird, wenn anhand der Benutzerkennung festgestellt wird, dass der Benutzer zugriffsberechtigt ist.

Gemäß einer Ausführungsform kann die Passwortanfrage ferner eine einem Benutzer des Benutzergeräts eindeutig zugeordnete Benutzerkennung umfassen. Beispielsweise kann die Benutzerkennung eine digitale Signatur zum eindeutigen Identifizieren des Benutzers gegenüber dem Entschlüsselungsgerät umfassen und/oder bestimmte Zugriffsrechte des Benutzers definieren. Anhand der Benutzerkennung kann geprüft werden, ob der Benutzer zugriffsberechtigt ist. Dementsprechend ist es möglich, dass die Passwortanfrage nur dann weiterverarbeitet wird oder das entschlüsselte Passwort nur dann generiert und/oder ans Benutzergerät gesendet wird, wenn der Benutzer zugriffsberechtigt ist.

Gemäß einer Ausführungsform kann das entschlüsselte Passwort unter Verwendung eines in einem Speicher des Entschlüsselungsgeräts gespeicherten privaten Schlüssels generiert werden. Der private Schlüssel kann mit einem öffentlichen Schlüssel zum Generieren des verschlüsselten Passworts - beispielsweise in einem asymmetrischen oder hybriden Verschlüsselungsverfahren - ein Schlüsselpaar bilden (siehe weiter oben).

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt eine Vorrichtung zur Datenverarbeitung gemäß einer Ausführungsform der Erfindung.
Fig. 2 zeigt ein Ablaufdiagramm zur Veranschaulichung eines Verfahrens gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

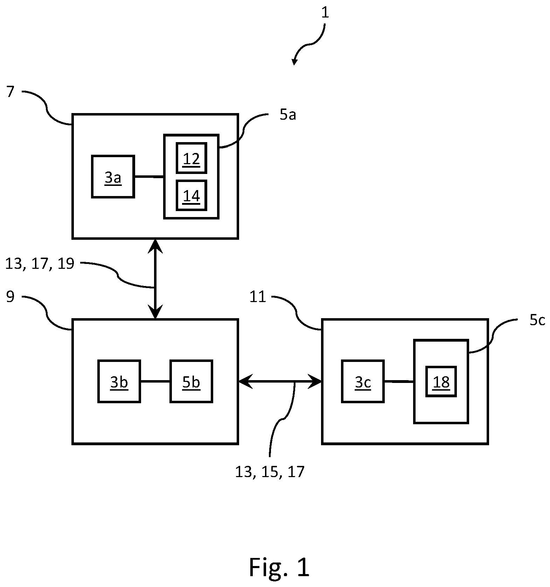

Fig. 1 zeigt beispielhaft eine Vorrichtung 1 zur Datenverarbeitung, die einen ersten Prozessor 3a, eine zweiten Prozessor 3b, einen dritten Prozessor 3c, einen an den ersten Prozessor 3a angebundenen ersten Speicher 5a, einen an den zweiten Prozessor 3b angebundenen zweiten Speicher 5b und einen an den dritten Prozessor 3c angebundenen dritten Speicher 5c umfasst.

Der erste Prozessor 3a und der erste Speicher 5a sind Komponenten eines medizintechnischen Geräts 7, hier eines Beatmungsgeräts 7 zum invasiven und/oder nicht invasiven, beispielsweise druck- und/oder fluss- und/oder volumengesteuerten Beatmen eines Patienten.

Der zweite Prozessor 3b und der zweite Speicher 5b sind Komponenten eines Benutzergeräts 9, beispielsweise eines PCs, eines Servers, eines Laptops, eines Tablets oder eines Smartphones.

Der dritte Prozessor 3c und der dritte Speicher 5c sind Komponenten eines Entschlüsselungsgeräts 11, beispielsweise eines weiteren PCs, eines weiteren Servers, eines weiteren Laptops, eines weiteren Tablets oder eines weiteren Smartphones.

Das Benutzergerät 9 kann mit dem Beatmungsgerät 7 und dem Entschlüsselungsgerät 11 jeweils über eine drahtlose und/oder drahtgebundene Datenkommunikationsverbindung und/oder über das Internet zur Datenkommunikation verbunden sein. Je nach verwendetem Datenkommunikationsprotokoll kann die Datenkommunikation zwischen dem Benutzergerät 9 und dem Beatmungsgerät 7 und/oder zwischen dem Benutzergerät 9 und dem Entschlüsselungsgerät 11 zusätzlich verschlüsselt sein.

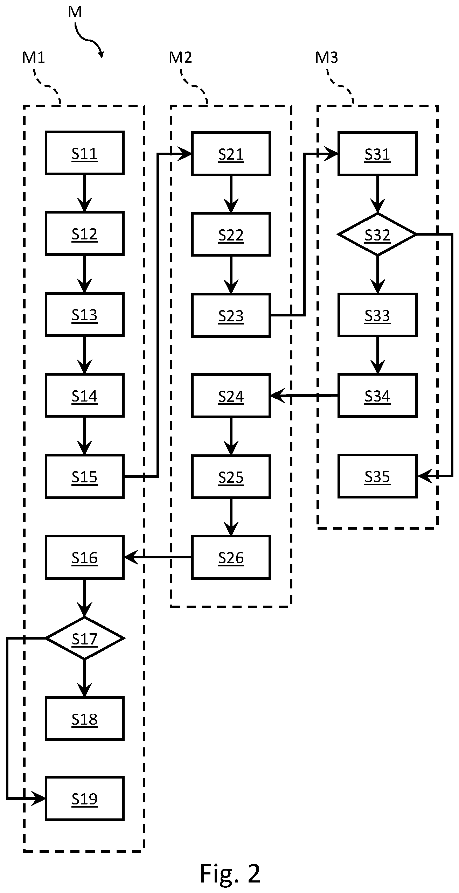

Die Vorrichtung 1 kann konfiguriert sein, um durch Ausführen eines entsprechenden Computerprogramms ein Verfahren M (siehe Fig. 2) zum Steuern eines Benutzerzugriffs auf das Beatmungsgerät 7 über das Benutzergerät 9 auszuführen.

In diesem Beispiel umfasst das Verfahren M Schritte S11 bis S19 eines ersten Verfahrens M1 zum Ermöglichen des Benutzerzugriffs, Schritte S21 bis S26 eines zweiten Verfahrens M2 zum Anfordern des Benutzerzugriffs und Schritte S31 bis S35 eines dritten Verfahrens M3 zum Betreiben des Entschlüsselungsgeräts 11.

Der erste Prozessor 3a kann konfiguriert sein, um das erste Verfahren M1 durch Ausführen eines im ersten Speicher 5a gespeicherten ersten Satzes von Befehlen des Computerprogramms auszuführen. In entsprechender Weise kann der zweite Prozessor 3b konfiguriert sein, um das zweite Verfahren M2 durch Ausführen eines im zweiten Speicher 5b gespeicherten zweiten Satzes von Befehlen des Computerprogramms auszuführen, und der dritte Prozessor 3c kann konfiguriert sein, um das dritte Verfahren M3 durch Ausführen eines im dritten Speicher 5c gespeicherten dritten Satzes von Befehlen des Computerprogramms auszuführen.

Nachstehend wird ein Beispiel für einen möglichen Ablauf des Verfahrens M beschrieben.

Im Schritt S11 wird durch den ersten Prozessor 3a ein zu verschlüsselndes (beispielsweise unverschlüsseltes) Passwort 12 generiert. Dazu kann beispielsweise mit einem Zufallsgenerator eine zufällige Zeichenfolge generiert und als das zu verschlüsselnde Passwort 12 im ersten Speicher 5a gespeichert werden.

Im Schritt S12 wird ein Timer als Reaktion auf das Generieren des zu verschlüsselnden Passworts 12 im Schritt S11 gestartet.

Wenn der Timer abgelaufen ist, beispielsweise nach 1, 2, 5 oder 10 Tagen, wird im Schritt S13 bestimmt, dass eine aktuelle Version des zu verschlüsselnden Passworts 12 ungültig ist. Hierauf kann automatisch eine neue Version des zu verschlüsselnden Passworts 12 generiert werden, die dann statt der aktuellen Version gültig ist. Beispielsweise kann die aktuelle Version mit der neuen Version überschrieben werden.

Beim Generieren der neuen Version kann beispielsweise der Timer zurückgesetzt und erneut gestartet werden, wobei die neue Version so lange gültig bleibt, bis der Timer erneut abgelaufen ist. Dies kann mit jedem neu generierten Passwort fortlaufend wiederholt werden, um eine automatische Erneuerung des zu verschlüsselnden Passworts 12 in regelmäßigen Abständen zu ermöglichen.

Zusätzlich oder alternativ kann das zu verschlüsselnde Passwort 12 jedes Mal neu generiert werden, wenn das Beatmungsgerät 7 ein- oder ausgeschaltet wird oder zwischen einem Stand-by-Betrieb und einem normalen Betrieb umschaltet.

Im Schritt S14 generiert der erste Prozessor 3a ein verschlüsseltes Passwort 13 durch Verschlüsseln des zu verschlüsselnden Passworts 12. Das Verschlüsseln kann beispielsweise in einem asymmetrischen oder hybriden Verschlüsselungsverfahren unter Verwendung eines öffentlichen Schlüssels 14 erfolgen. Der öffentliche Schlüssel 14 kann im ersten Speicher 5a gespeichert sein.

Im Schritt S15 wird das verschlüsselte Passwort 13 ans Benutzergerät 9 gesendet, beispielsweise über eine UART- oder USB-Schnittstelle.

Im Schritt S21 wird das verschlüsselte Passwort 13 im Benutzergerät 9 empfangen.

Im Schritt S22 generiert der zweite Prozessor 3b eine das verschlüsselte Passwort 13 umfassende Passwortanfrage 15.

Im Schritt S23 wird die Passwortanfrage 15 ans Entschlüsselungsgerät 11 gesendet, beispielsweise über das Internet und/oder ein lokales Netzwerk.

Im Schritt S31 wird die Passwortanfrage 15 im Entschlüsselungsgerät 11 empfangen.

Es ist möglich, dass die Passwortanfrage 15 zusätzlich zum verschlüsselten Passwort 13 eine einem jeweiligen Benutzer des Benutzergeräts 9 eindeutig zugeordnete Benutzerkennung umfasst. In diesem Fall kann im Schritt S32 anhand der Benutzerkennung geprüft werden, ob der Benutzer zugriffsberechtigt ist.

Nur wenn im Schritt S32 festgestellt wird, dass der Benutzer zugriffsberechtigt ist, generiert der dritte Prozessor 3c im Schritt S33 ein entschlüsseltes Passwort 17 durch Entschlüsseln des verschlüsselten Passworts 13. Andernfalls wird im Schritt S35 das Entschlüsseln verweigert. Beispielsweise kann der Benutzer dann zugriffsberechtigt sein, wenn er anhand der Benutzerkennung erfolgreich durch das Entschlüsselungsgerät 11 authentifiziert werden konnte.

Es ist möglich, dass das entschlüsselte Passwort 17 im Schritt S33 unter Verwendung eines im dritten Speicher 5c gespeicherten privaten Schlüssels 18 generiert wird. Der private Schlüssel 18 kann mit dem öffentlichen Schlüssel 14 zum Generieren des verschlüsselten Passworts 13 im Schritt S14 ein Schlüsselpaar bilden.

Im Schritt S34 wird das entschlüsselte Passwort 17 ans Benutzergerät 9 gesendet, beispielsweise ebenfalls über das Internet und/oder das lokale Netzwerk.

Im Schritt S24 wird das entschlüsselte Passwort 17 im Benutzergerät 9 empfangen.

Im Schritt S25 generiert der zweite Prozessor 3b eine das entschlüsselte Passwort 17 umfassende Zugriffsanfrage 19.

Im Schritt S26 wird die Zugriffsanfrage 19 ans Beatmungsgerät 7 gesendet, beispielsweise über das Internet und/oder ein lokales Netzwerk.

Im Schritt S16 wird die Zugriffsanfrage 19 im Beatmungsgerät 7 empfangen.

Im Schritt S17 wird geprüft, ob das entschlüsselte Passwort 17 mit dem zu verschlüsselnden Passwort 12 übereinstimmt, d. h., ob das entschlüsselte Passwort 17 korrekt ist.

Nur wenn das entschlüsselte Passwort 17 korrekt ist, wird im Schritt S18 der Benutzerzugriff auf das Beatmungsgerät 7 - beispielsweise ein Root-Zugriff auf dessen Betriebssystem - gewährt, sodass der jeweilige Benutzer vom Benutzergerät 9 aus beispielsweise eine Hard- und/oder Softwarekonfiguration des Beatmungsgeräts 7 ändern und/oder bestimmte Funktionen des Beatmungsgeräts 7 testen kann. Andernfalls wird im Schritt S19 der Benutzerzugriff verweigert.

Es ist möglich, dass die Zugriffsanfrage 19 zusätzlich zum entschlüsselten Passwort 17 eine dem jeweiligen Benutzer eindeutig zugeordnete Benutzerkennung umfasst. In diesem Fall kann im Schritt S17 anhand der Benutzerkennung zusätzlich geprüft werden, ob der Benutzer zugriffsberechtigt ist. Dementsprechend wird der Benutzerzugriff im Schritt S18 nur unter der Voraussetzung ermöglicht, dass der Benutzer zugriffsberechtigt ist. Beispielsweise kann der Benutzer dann zugriffsberechtigt sein, wenn er anhand der Benutzerkennung erfolgreich durch das Beatmungsgerät 7 authentifiziert werden konnte.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Vorrichtung zur Datenverarbeitung
- 3a: erster Prozessor
- 3b: zweiter Prozessor
- 3c: dritter Prozessor
- 5a: erster Speicher
- 5b: zweiter Speicher
- 5c: dritter Speicher
- 7: medizintechnisches Gerät, Beatmungsgerät
- 9: Benutzergerät
- 11: Entschlüsselungsgerät
- 12: zu verschlüsselndes Passwort
- 13: verschlüsseltes Passwort
- 14: öffentlicher Schlüssel
- 15: Passwortanfrage
- 17: entschlüsseltes Passwort
- 18: privater Schlüssel
- 19: Zugriffsanfrage
- M: Verfahren zum Steuern eines Benutzerzugriffs auf das medizintechnische Gerät
- M1: erstes Verfahren (zum Ermöglichen des Benutzerzugriffs)
- M2: zweites Verfahren (zum Anfordern des Benutzerzugriffs)
- M3: drittes Verfahren (zum Betreiben des Entschlüsselungsgeräts)
- S1...: Schritte des ersten Verfahrens
- S2...: Schritte des zweiten Verfahrens
- S3...: Schritte des dritten Verfahrens

## Patentansprüche

1. Verfahren (M1) zum Ermöglichen eines Benutzerzugriffs auf ein medizintechnisches Gerät (7) über ein Benutzergerät (9), wobei das Verfahren (M1) durch einen Prozessor (3a) des medizintechnischen Geräts (7) ausgeführt wird und umfasst:
Generieren (S14) eines verschlüsselten Passworts (13) durch Verschlüsseln eines zu verschlüsselnden Passworts (12);
Senden (S15) des verschlüsselten Passworts (13) ans Benutzergerät (9), um ein Entschlüsseln des verschlüsselten Passworts (13) zu ermöglichen;
Empfangen (S16) einer vom Benutzergerät (9) gesendeten Zugriffsanfrage (19) zum Anfordern des Benutzerzugriffs, wobei die Zugriffsanfrage (19) ein entschlüsseltes Passwort (17) umfasst;
Prüfen (S17), ob das entschlüsselte Passwort (17) mit dem zu verschlüsselnden Passwort (12) übereinstimmt;
wenn das entschlüsselte Passwort (17) mit dem zu verschlüsselnden Passwort (12) übereinstimmt: Ermöglichen (S18) des Benutzerzugriffs.

2. Verfahren (M1) nach Anspruch 1,
wobei das verschlüsselte Passwort (13) unter Verwendung eines in einem Speicher (5a) des medizintechnischen Geräts (7) gespeicherten öffentlichen Schlüssels (14) in einem asymmetrischen oder hybriden Verschlüsselungsverfahren generiert wird, wobei der öffentliche Schlüssel (14) mit einem privaten Schlüssel (18) zum Generieren (S33) des entschlüsselten Passworts (17) ein Schlüsselpaar bildet.

3. Verfahren (M1) nach einem der vorhergehenden Ansprüche, ferner umfassend:
Generieren (S11) des zu verschlüsselnden Passworts (12).

4. Verfahren (M1) nach Anspruch 3, ferner umfassend:
Starten (S12) eines Timers als Reaktion auf das Generieren (S11) des zu verschlüsselnden Passworts (12);
wenn der Timer abgelaufen ist:
Generieren (S13) einer neuen Version des zu verschlüsselnden Passworts (12);
Überschreiben (S13) einer aktuellen Version des zu verschlüsselnden Passworts (12) mit der neuen Version.

5. Verfahren (M1) nach Anspruch 3 oder 4,
wobei das zu verschlüsselnde Passwort (12) jedes Mal neu generiert wird, wenn das medizintechnische Gerät (7) ein- oder ausgeschaltet wird oder zwischen verschiedenen Betriebsarten zum Betreiben des medizintechnischen Geräts (7), insbesondere einem Stand-by-Betrieb und einem normalen Betrieb, umschaltet; und/oder
wobei das zu verschlüsselnde Passwort (12) unter Verwendung eines Zufallsgenerators zum Generieren einer zufälligen Zeichenfolge generiert wird.

6. Verfahren (M1) nach einem der vorhergehenden Ansprüche,
wobei die Zugriffsanfrage (19) ferner eine einem Benutzer des Benutzergeräts (9) eindeutig zugeordnete Benutzerkennung umfasst;
wobei anhand der Benutzerkennung geprüft wird, ob der Benutzer zugriffsberechtigt ist;
wobei der Benutzerzugriff nur dann ermöglicht wird, wenn der Benutzer zugriffsberechtigt ist.

7. Verfahren (M2) zum Anfordern eines Benutzerzugriffs auf ein medizintechnisches Gerät (7) über ein Benutzergerät (9), wobei das Verfahren (M2) durch einen Prozessor (3b) des Benutzergeräts (9) ausgeführt wird und umfasst:
Empfangen (S21) eines vom medizintechnischen Gerät (7) gesendeten verschlüsselten Passworts (13);
Generieren (S22) einer das verschlüsselte Passwort (13) umfassenden Passwortanfrage (15) zum Anfordern eines entschlüsselten Passworts (17);
Senden (S23) der Passwortanfrage (15) an ein Entschlüsselungsgerät (11) zum Entschlüsseln des verschlüsselten Passworts (13);
Empfangen (S24) eines vom Entschlüsselungsgerät (11) gesendeten entschlüsselten Passworts (17);
Generieren (S25) einer das entschlüsselte Passwort (17) umfassenden Zugriffsanfrage (19) zum Anfordern des Benutzerzugriffs;
Senden (S26) der Zugriffsanfrage (19) ans medizintechnische Gerät (7).

8. Verfahren (M3) zum Betreiben eines Entschlüsselungsgeräts (11), wobei das Verfahren (M3) durch einen Prozessor (3c) des Entschlüsselungsgeräts (11) ausgeführt wird und umfasst:
Empfangen (S31) einer von einem Benutzergerät (9) gesendeten Passwortanfrage (15) zum Anfordern eines entschlüsselten Passworts (17), wobei die Passwortanfrage (15) ein verschlüsseltes Passwort (13) umfasst, wobei das verschlüsselte Passwort (13) durch Verschlüsseln eines zu verschlüsselnden Passworts (12) durch einen Prozessor (3a) eines medizintechnischen Geräts (7) generiert wurde;
Generieren (S33) des entschlüsselten Passworts (17) durch Entschlüsseln des verschlüsselten Passworts (13);
Senden (S34) des entschlüsselten Passworts (17) ans Benutzergerät (9).

9. Verfahren (M3) nach Anspruch 8,
wobei die Passwortanfrage (15) ferner eine einem Benutzer des Benutzergeräts (9) eindeutig zugeordnete Benutzerkennung umfasst;
wobei anhand der Benutzerkennung geprüft wird, ob der Benutzer zugriffsberechtigt ist;
wobei das entschlüsselte Passwort (17) nur dann generiert und/oder ans Benutzergerät (9) gesendet wird, wenn der Benutzer zugriffsberechtigt ist.

10. Verfahren (M3) nach Anspruch 8 oder 9,
wobei das entschlüsselte Passwort (17) unter Verwendung eines in einem Speicher (5c) des Entschlüsselungsgeräts (11) gespeicherten privaten Schlüssels (18) generiert wird, wobei der private Schlüssel (18) mit einem öffentlichen Schlüssel (14) zum Generieren (S14) des verschlüsselten Passworts (13) in einem asymmetrischen oder hybriden Verschlüsselungsverfahren ein Schlüsselpaar bildet.

11. Verfahren (M) zum Steuern eines Benutzerzugriffs auf ein medizintechnisches Gerät (7) über ein Benutzergerät (9), wobei das Verfahren (M) umfasst:
die Schritte (S11, S12, S13, S14, S15, S16, S17, S18, S19) des Verfahrens (M1) nach einem der Ansprüche 1 bis 6;
die Schritte (S21, S22, S23, S24, S25, S26) des Verfahrens (M2) nach Anspruch 7.

12. Verfahren (M) nach Anspruch 11, ferner umfassend:
die Schritte (S31, S32, S33, S34, S35) des Verfahrens (M3) nach einem der Ansprüche 8 bis 10.

13. Vorrichtung (1) zur Datenverarbeitung, umfassend:
einen ersten Prozessor (3a), der konfiguriert ist, um das Verfahren (M1) nach einem der Ansprüche 1 bis 6 auszuführen; und/oder
einen zweiten Prozessor (3b), der konfiguriert ist, um das Verfahren (M2) nach Anspruch 7 auszuführen; und/oder
einen dritten Prozessor (3c), der konfiguriert ist, um das Verfahren (M3) nach einem der Ansprüche 8 bis 10 auszuführen.

14. Computerprogramm, umfassend:
einen ersten Satz von Befehlen, die die Vorrichtung (1) nach Anspruch 13 beim Ausführen des Computerprogramms durch die Vorrichtung (1) veranlassen, das Verfahren (M1) nach einem der Ansprüche 1 bis 6 auszuführen; und/oder
einen zweiten Satz von Befehlen, die die Vorrichtung (1) nach Anspruch 13 beim Ausführen des Computerprogramms durch die Vorrichtung (1) veranlassen, das Verfahren (M2) nach Anspruch 7 auszuführen; und/oder
einen dritten Satz von Befehlen, die die Vorrichtung (1) nach Anspruch 13 beim Ausführen des Computerprogramms durch die Vorrichtung (1) veranlassen, das Verfahren (M3) nach einem der Ansprüche 8 bis 10 auszuführen.

15. Computerlesbares Medium, auf dem das Computerprogramm nach Anspruch 14 gespeichert ist.

## Geänderte Patentansprüche

### Geänderte Patentansprüche gemäss Regel 137(2) EPÜ.

1. Verfahren (M1) zum Ermöglichen eines Benutzerzugriffs auf ein medizintechnisches Gerät (7) über ein Benutzergerät (9), wobei das Verfahren (M1) durch einen Prozessor (3a) des medizintechnischen Geräts (7) ausgeführt wird und umfasst:
Generieren (S14) eines verschlüsselten Passworts (13) durch Verschlüsseln eines zu verschlüsselnden Passworts (12) unter Verwendung eines in einem Speicher (5a) des medizintechnischen Geräts (7) gespeicherten öffentlichen Schlüssels (14) in einem asymmetrischen Verschlüsselungsverfahren, wobei der öffentliche Schlüssel (14) mit einem privaten Schlüssel (18) zum Generieren (S33) eines entschlüsselten Passworts (17) ein Schlüsselpaar bildet, wobei das asymmetrische Verschlüsselungsverfahren ein Post-Quanten-Algorithmus ist;
Senden (S15) des verschlüsselten Passworts (13) ans Benutzergerät (9), um ein Entschlüsseln des verschlüsselten Passworts (13) zu ermöglichen;
Empfangen (S16) einer vom Benutzergerät (9) gesendeten Zugriffsanfrage (19) zum Anfordern des Benutzerzugriffs, wobei die Zugriffsanfrage (19) das entschlüsselte Passwort (17) umfasst;
Prüfen (S17), ob das entschlüsselte Passwort (17) mit dem zu verschlüsselnden Passwort (12) übereinstimmt;
wenn das entschlüsselte Passwort (17) mit dem zu verschlüsselnden Passwort (12) übereinstimmt: Ermöglichen (S18) des Benutzerzugriffs.

2. Verfahren (M1) nach Anspruch 1, ferner umfassend:
Generieren (S11) des zu verschlüsselnden Passworts (12).

3. Verfahren (M1) nach Anspruch 2, ferner umfassend:
Starten (S12) eines Timers als Reaktion auf das Generieren (S11) des zu verschlüsselnden Passworts (12);
wenn der Timer abgelaufen ist:
Generieren (S13) einer neuen Version des zu verschlüsselnden Passworts (12);
Überschreiben (S13) einer aktuellen Version des zu verschlüsselnden Passworts (12) mit der neuen Version.

4. Verfahren (M1) nach Anspruch 2 oder 3,
wobei das zu verschlüsselnde Passwort (12) jedes Mal neu generiert wird, wenn das medizintechnische Gerät (7) ein- oder ausgeschaltet wird oder zwischen verschiedenen Betriebsarten zum Betreiben des medizintechnischen Geräts (7), insbesondere einem Stand-by-Betrieb und einem normalen Betrieb, umschaltet; und/oder
wobei das zu verschlüsselnde Passwort (12) unter Verwendung eines Zufallsgenerators zum Generieren einer zufälligen Zeichenfolge generiert wird.

5. Verfahren (M1) nach einem der vorhergehenden Ansprüche,
wobei die Zugriffsanfrage (19) ferner eine einem Benutzer des Benutzergeräts (9) eindeutig zugeordnete Benutzerkennung umfasst;
wobei anhand der Benutzerkennung geprüft wird, ob der Benutzer zugriffsberechtigt ist;
wobei der Benutzerzugriff nur dann ermöglicht wird, wenn der Benutzer zugriffsberechtigt ist.

6. Verfahren (M2) zum Anfordern eines Benutzerzugriffs auf ein medizintechnisches Gerät (7) über ein Benutzergerät (9), wobei das Verfahren (M2) durch einen Prozessor (3b) des Benutzergeräts (9) ausgeführt wird und umfasst:
Empfangen (S21) eines vom medizintechnischen Gerät (7) gesendeten verschlüsselten Passworts (13), wobei das verschlüsselte Passwort (13) unter Verwendung eines in einem Speicher (5a) des medizintechnischen Geräts (7) gespeicherten öffentlichen Schlüssels (14) in einem asymmetrischen Verschlüsselungsverfahren durch einen Prozessor (3a) des medizintechnischen Geräts (7) generiert wurde, wobei das asymmetrische Verschlüsselungsverfahren ein Post-Quanten-Algorithmus ist;
Generieren (S22) einer das verschlüsselte Passwort (13) umfassenden Passwortanfrage (15) zum Anfordern eines entschlüsselten Passworts (17);
Senden (S23) der Passwortanfrage (15) an ein Entschlüsselungsgerät (11) zum Entschlüsseln des verschlüsselten Passworts (13) unter Verwendung eines in einem Speicher (5c) des Entschlüsselungsgeräts (11) gespeicherten privaten Schlüssels (18), der mit dem öffentlichen Schlüssel (14) ein Schlüsselpaar bildet;
Empfangen (S24) eines vom Entschlüsselungsgerät (11) gesendeten entschlüsselten Passworts (17);
Generieren (S25) einer das entschlüsselte Passwort (17) umfassenden Zugriffsanfrage (19) zum Anfordern des Benutzerzugriffs;
Senden (S26) der Zugriffsanfrage (19) ans medizintechnische Gerät (7).

7. Verfahren (M3) zum Betreiben eines Entschlüsselungsgeräts (11), wobei das Verfahren (M3) durch einen Prozessor (3c) des Entschlüsselungsgeräts (11) ausgeführt wird und umfasst:
Empfangen (S31) einer von einem Benutzergerät (9) gesendeten Passwortanfrage (15) zum Anfordern eines entschlüsselten Passworts (17), wobei die Passwortanfrage (15) ein verschlüsseltes Passwort (13) umfasst, wobei das verschlüsselte Passwort (13) durch Verschlüsseln eines zu verschlüsselnden Passworts (12) durch einen Prozessor (3a) eines medizintechnischen Geräts (7) unter Verwendung eines in einem Speicher (5a) des medizintechnischen Geräts (7) gespeicherten öffentlichen Schlüssels (14) in einem asymmetrischen Verschlüsselungsverfahren generiert wurde, wobei das asymmetrische Verschlüsselungsverfahren ein Post-Quanten-Algorithmus ist;
Generieren (S33) des entschlüsselten Passworts (17) durch Entschlüsseln des verschlüsselten Passworts (13) unter Verwendung eines in einem Speicher (5c) des Entschlüsselungsgeräts (11) gespeicherten privaten Schlüssels (18), der mit dem öffentlichen Schlüssel (14) ein Schlüsselpaar bildet;
Senden (S34) des entschlüsselten Passworts (17) ans Benutzergerät (9).

8. Verfahren (M3) nach Anspruch 7,
wobei die Passwortanfrage (15) ferner eine einem Benutzer des Benutzergeräts (9) eindeutig zugeordnete Benutzerkennung umfasst;
wobei anhand der Benutzerkennung geprüft wird, ob der Benutzer zugriffsberechtigt ist;
wobei das entschlüsselte Passwort (17) nur dann generiert und/oder ans Benutzergerät (9) gesendet wird, wenn der Benutzer zugriffsberechtigt ist.

9. Verfahren (M) zum Steuern eines Benutzerzugriffs auf ein medizintechnisches Gerät (7) über ein Benutzergerät (9), wobei das Verfahren (M) umfasst:
die Schritte (S11, 512, S13, S14, S15, S16, S17, S18, S19) des Verfahrens (M1) nach einem der Ansprüche 1 bis 5;
die Schritte (S21, S22, S23, S24, S25, S26) des Verfahrens (M2) nach Anspruch 6.

10. Verfahren (M) nach Anspruch 9, ferner umfassend:
die Schritte (S31, S32, S33, S34, S35) des Verfahrens (M3) nach Anspruch 7 oder 8.

11. Vorrichtung (1) zur Datenverarbeitung, umfassend:
einen ersten Prozessor (3a), der konfiguriert ist, um das Verfahren (M1) nach einem der Ansprüche 1 bis 5 auszuführen; und/oder
einen zweiten Prozessor (3b), der konfiguriert ist, um das Verfahren (M2) nach Anspruch 6 auszuführen; und/oder
einen dritten Prozessor (3c), der konfiguriert ist, um das Verfahren (M3) nach Anspruch 7 oder 8 auszuführen.

12. Computerprogramm, umfassend:
einen ersten Satz von Befehlen, die die Vorrichtung (1) nach Anspruch 11 beim Ausführen des Computerprogramms durch die Vorrichtung (1) veranlassen, das Verfahren (M1) nach einem der Ansprüche 1 bis 5 auszuführen; und/oder
einen zweiten Satz von Befehlen, die die Vorrichtung (1) nach Anspruch 11 beim Ausführen des Computerprogramms durch die Vorrichtung (1) veranlassen, das Verfahren (M2) nach Anspruch 6 auszuführen; und/oder
einen dritten Satz von Befehlen, die die Vorrichtung (1) nach Anspruch 11 beim Ausführen des Computerprogramms durch die Vorrichtung (1) veranlassen, das Verfahren (M3) nach Anspruch 7 oder 8 auszuführen.

13. Computerlesbares Medium, auf dem das Computerprogramm nach Anspruch 12 gespeichert ist.
